# EUROPÄISCHE PATENTSCHRIFT

(11) **EP 0 879 028 B1**
(45) Veröffentlichungstag und Bekanntmachung des Hinweises auf die Patenterteilung: **27.10.1999**
(21) Anmeldenummer: 97904286.8
(22) Anmeldetag: 06.02.1997
(51) Int. Cl.: A61F 2/06

(54) **GEFÄSSPROTHESE**
VASCULAR PROSTHESIS
PROTHESE VASCULAIRE

(30) Priorität: 06.02.1996 AT 21896
(43) Veröffentlichungstag der Anmeldung: 25.11.1998
(73) Patentinhaber: Havel, Michael, 1140 Wien (AT); Ehrlich, Marek, 1030 Wien (AT)
(72) Erfinder: Havel, Michael, 1140 Wien (AT); Ehrlich, Marek, 1030 Wien (AT)
(74) Vertreter: Haffner, Thomas M., Dr.
(86) Internationale Anmeldenummer: AT9700022
(87) Internationale Veröffentlichungsnummer: WO9728758

(56) Entgegenhaltungen:
- EP-A- 0 364 420
- EP-A- 0 472 731
- EP-A- 0 539 237
- US-A- 5 078 720
- US-A- 5 415 664

## Beschreibung

Die Erfindung bezieht sich auf eine Gefäßprothese zum Einsatz in pathologisch veränderte Aortenabschnitte mit einem Transportbehälter sowie einer aus diesem Transportbehälter ausschiebbaren radial aufweitbaren Prothese, wobei der Boden des Transportbehälters mit einem Vorschubdraht in Zug- und Druckrichtung kraftschlüssig verbunden ist, wie sie beispielsweise aus der US-A-5415664 bekannt ist.

Trotz der großen Fortschritte, welche die Gefäßchirurgie in der jüngeren Vergangenheit machen konnte, stellen die Aortendissektion (Zerschichtung der Aortenwand) bzw. das atherosklerotische Aortenaneurysma (Erweiterung des Aortengefäßkalibers) im Bereich der absteigenden Aorta (Aorta descendens) immer noch einen großen herzchirurgischen Eingriff dar, welcher mit hoher Morbilität und Letalität verbunden ist.

Die bisherige konventionelle Methode war der chirurgische Ersatz der pathologisch veränderten Aorta, sei es mittels Hypothermie und Kreislaufstillstand oder durch proximales und distales Klemmen der erweiterten Aorta oder durch Verwendung eines Links-herzbypasses. Diese Methoden sind einerseits mit relativ langen Krankenhausaufenthalten bzw. mit großen finanziellen Kosten verbunden, andererseits betrifft diese Erkrankung vor allem ein älteres Patientenkollektiv (Durchschnittsalter 60 Jahre) mit erhöhtem Operationsrisiko. Um das Operationsrisiko zu minimieren, sind bereits neue Operationsarten bekannt geworden. Bei diesen Operationstechniken wird in der Leistengegend die Arterie punktiert und eine Prothese, die sich in einer Transportkapsel befindet, in die erweiterte Aorta vorgeschoben, positioniert und anschließend die Transportkapsel zurückgezogen. In diesem Moment entfaltet sich die zusammengerollte Prothese und kommt im Bereich der zu behandelnden Stelle zu liegen. Durch den relativ kleinen Gefäßdurchmesser der Leistenarterie ist die Einführung der Prothese nicht immer unproblematisch und in manchen Fällen sogar unmöglich. Eine einmal an den geschädigten Gefäßabschnitt im Thorax gebrachte Prothese kann daher im nachhinein nur noch bedingt nachpositioniert werden. Um einen bei der Einpflanzung und Positionierung der Prothese eintretenden Notfall zu behandeln, muß zunächst der Brustkorb geöffnet werden, um dann die Prothese richtig zu positionieren und konventionell chirurgisch anzunähen. Während dieses schweren operativen Eingriffes vergeht jedoch relativ viel Zeit bis der Patient entsprechend präpariert ist und die für die Operation notwendigen Vorbereitungen durchgeführt sind und die notwendige Operationsumgebung geschaffen ist. Prinzipiell vergeht bei diesen Vorkehrungen wertvolle Zeit, was das operative Risiko erheblich ansteigen läßt.

Die vorliegende Erfindung zielt nun darauf ab, eine Möglichkeit zu schaffen, mit der pathologisch veränderte Aortenabschnitte durch Gefäßprothesen ersetzt werden, wobei das operative Risiko minimiert wird, die Operationszeit erheblich verkürzt und die Genauigkeit der Positionierung der Prothese verbessert werden kann.

Zur Lösung dieser Aufgabe wird die Gefäßprothese so ausgebildet, daß die Prothese mit Zugelementen, wie z.B. Fäden, verbunden ist und daß der Boden des Transportbehälters mit Durchbrechungen ausgebildet und/oder über radiale Arme mit dem Mantel des Transportbehälters verbunden ist. Der operative Eingriff setzt hier zwar die Öffnung des Thorax voraus, da die Prothese bevorzugt über eine Öffnung im Aortenbogen eingebracht und daher entgegen der Richtung der Einbringung bekannter Prothesen eingeführt wird. Eine gegebenenfalls erforderliche Notoperation ist aber nicht notwendig, da der Patient für diesen Eingriff vorbereitet wurde. Die Einbringung über den Aortenbogen kann aber nun rasch erfolgen und ist nicht durch engere Arterien, wie die eingangs genannte A. femoralis beeinträchtigt, welche bei großem Prothesendurchmesser lädiert werden könnte. Dadurch, daß nunmehr die Prothese selbst mit Zugelementen, wie z.B. Fäden, verbunden ist, läßt sie sich, nachdem sie in einem Transportbehälter an die vorgesehene Position gebracht worden ist, leicht aus diesem ausbringen, indem der Transportbehälter mittels eines Vorschubdrahtes weiterverschoben wird und die Prothese durch die Fäden an der gewünschten Position zurückgehlaten wird. Dadurch, daß die Prothese durch die unter Zug stehenden Fäden während des Vorschubes des Transportbehälters an Ort und Stelle gehalten wird, können sich die aus dem Behälter freiwerdenden Prothesenabschnitte schnell an der genau vorbestimmten Position entfalten und ausweiten. Dadurch, daß das Freiwerden der Prothese besonders leichtgängig geschieht, können unerwünscht hohe Reibungseffekte weitestgehend vermieden werden. Nachdem die Prothese aus dem Transportbehälter vollständig ausgebracht worden ist, kann letzterer durch die jetzt auf den Gefäßdurchmesser aufgeweitete Prothese wegen seines im Vergleich hierzu geringeren Durchmessers vom Vorschubdraht zur Operationsöffnung zurückgezogen werden. Während des Einbringvorganges dienen die Zugelemente nicht nur der Lokalisierung sondern erlauben überdies die Position der Prothese zu korrigieren. Nach erfolgter Positionierung der Prothese an der geschädigten Gefäßstelle und zurückgezogenem Transportbehälter werden die vier Fäden von der Prothese abgetrennt und entfernt. Die Verwendung einer derartigen Prothese schafft somit prinzipiell die Möglichkeit die geschädigten Aortabereiche durch die Aorta nach Öffnung des Brustkorbes und Defibrillierung des Herzens von oberhalb der Schadstelle (deszendent) aus der Nähe zu behandeln. Insgesamt gesehen kann somit die Operationszeit und das Operationsrisiko, welche beim Einbringen der Prothese und des Transportbehälters durch eine an der Leistenarterie vorgenommene Punktation erhebliche Schwierigkeiten bereiten, wesentlich gesenkt werden. Dadurch, daß der Boden des Transportbehälters mit Durchbrechungen ausgebildet und/oder über radiale Arme mit dem Mantel des Transportbehälters verbunden ist, werden die Öffnungen an der Stirnseite des Behälters bereitgestellt, sodaß gegebenenfalls auch während zum Stillstand gebrachten Blutflusses Blut den Behälter durchströmen kann. Dadurch wird verhindert, daß Restströmungen, Verwirbelungen oder Defibrillierungsströmungen die exakte Positionierung der Prothese behindern bzw. schlechtestenfalls erheblich lokal versetzen.

In einer besonders bevorzugten Ausführungsform ist der Mantel des Transportbehälters von axial orientierten Stäben gebildet.

Dadurch, daß der Mantel des Transportbehälters von axial orientierten Stäben gebildet ist, kann die Trennung des Transportbehälters von der Prothese besonders reibungsarm und somit leichtgängig ohne großen Kraftaufwand und noch schneller erfolgen. Durch diese Maßnahme wird insgesamt erreicht die Prothese weiter zu stabilisieren und nur kleinere Bereiche des geschädigten Gefäßabschnittes zu belasten.

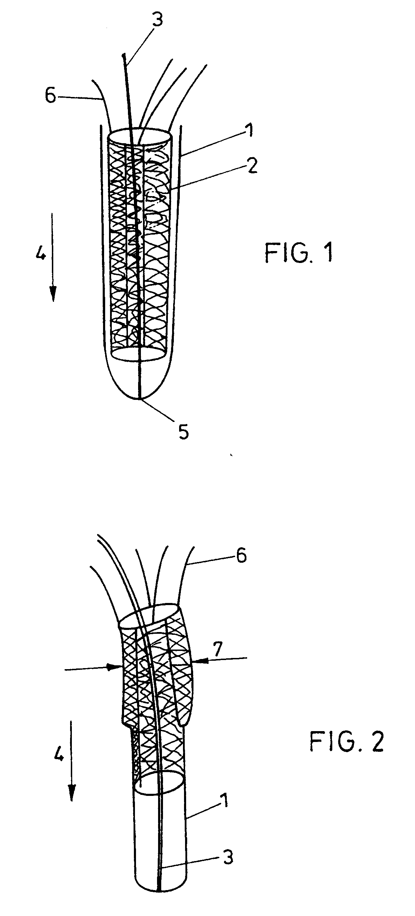

Die Erfindung wird nachfolgend anhand eines in Zeichnungen schematisch dargestellten Ausführungsbeispieles näher erläutert. In diesen zeigen Fig. 1 bis 3 Momentanaufnahmen der Prothese sowie des Transportbehälters während des Einbringens und Positionierens im geschädigten Gefäßabschnitt.

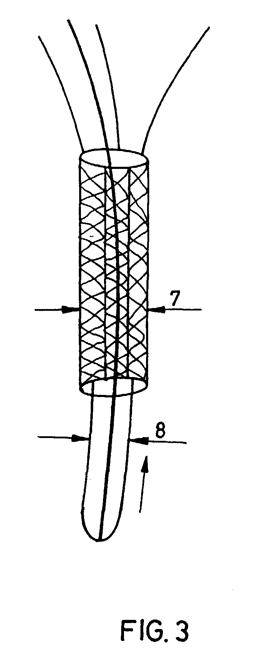

In Fig. 1 ist mit 1 ein als Zylinder ausgebildeter Transportbehälter bezeichnet, in welchem eine gefaltete oder gerollte Prothese 2 eingebracht ist. Durch den Transportbehälter 1 und die Prothese 2 ist ein Vorschubdraht 3 geführt, welcher am in Einführrichtung entsprechend dem Pfeil 4 weisenden Transportbehälterboden 5 befestigt ist. Die Prothese weist Zugelemente 6 auf, welche bis zum Erreichen des Bestimmungsortes der Gefäßprothese entspannt sind. Fig. 2 zeigt den Transportbehälter und die Prothese zu einem Zeitpunkt, wenn die Prothese den vorbestimmten und behandlungsbedürftigen Gefäßteil bereits erreicht hat. Die Fäden 6 werden jetzt beim weiteren Vorschub des Transportbehälters 1 mittels des Vorschubdrahtes 3 in Richtung des Pfeiles 4 auf Zug belastet, wodurch die Prothese nach und nach freikommt und sich entsprechend dem erforderlichen Durchmesser 7 bzw. der Gefäßwand aufweitet. Fig. 3 stellt den Zeitpunkt der vollständig aus dem Behälter 1 ausgetretenen Prothese 2 dar. Die Prothese 2 hat nun über die gesamte Länge den dem Gefäßdurchmesser entsprechenden Prothesedurchmesser 7. Dieser ist größer als der Durchmesser 8 des Behälters 1, wodurch ab diesem Zeitpunkt die Bewegungsumkehr des Behälters 1 entgegen der Richtung des Pfeiles 4 möglich wird. Die Bewegung des Behälters durch das Lumen der Prothese 7 wird nunmehr durch Zug am Draht 3 erreicht.

## Patentansprüche

1. Gefäßprothese zum Einsatz in pathologisch veränderte Aortenabschnitte mit einem Transportbehälter (1) sowie einer aus diesem Transportbehälter (1) ausschiebbaren radial aufweitbaren Prothese (2), wobei der Boden (5) des Transportbehälters (1) mit einem Vorschubdraht (3) in Zug- und Druckrichtung kraftschlüssig verbunden ist, dadurch gekennzeichnet, daß die Prothese (2) mit Zugelementen (6), wie z.B. Fäden, verbunden ist und daß der Boden (5) des Transportbehälters (1) mit Durchbrechungen ausgebildet ist und/oder über radiale Arme mit dem Mantel des Transportbehälters (1) verbunden ist.

2. Gefäßprothese nach Anspruch 1, dadurch gekennzeichnet, daß der Mantel des Transportbehälters (1) von axial orientierten Stäben gebildet ist.

## Claims

1. Vascular prosthesis assembly used in pathologically changed aortic sections comprising a transport container (1) as well as a radially-expandable prosthesis (2) removable from this transport container (1), the bottom (5) of the transport conatiner (1) beeing frictionally, in the traction and compressing direction, connected with a feed wire (3), characterized in that the prosthesis is connected with traction elements (6), such as threads, and that the bottom (5) of the transport container (1) is perforated and/or connected with the wall of the transport container (1) via radial arms.

2. Vascular prosthesis assembly according to claim 1, characterized in that the wall of the transport container (1) is formed by bars aligned in axial direction.

## Revendications

1. Prothèse vasculaire pour être utilisé dans des aortes pathologiquement transformées comportant un récipient de transport (1) et une prothèse (2) capable d'être élargie en direction radiale et d'être glissée vers le dehors du récipient de transport (1), le fond (5) du récipient de transport (1) êtant relié, entrainé par adhérence en direction de traction et de pression, à un fil de fer d'avance caractérisée en ce que la prothèse (2) est reliée à d'elements de traction, par example des fils, et en ce que le fond (5) du récipient de transport (1) est formé avec des découpures et/ou est relié en manteau du récipient de transport (1) à travers de bras radials.

2. Prothèse selon la revendication 1, caractérisée en ce que le manteau du récipient de transport (1) est formé de barres orientés en direction axial.
